Europäisches Patentamt

European Patent Office

Office européen des brevets

(11) Publication number: **0 630 859 A1**

# EUROPEAN PATENT APPLICATION

(21) Application number: **93870116.6**

(22) Date of filing: **24.06.93**

(51) Int. Cl.5: **C02F 3/04**, C12N 11/08

(43) Date of publication of application:
**28.12.94 Bulletin 94/52**

(84) Designated Contracting States:
**BE**

(71) Applicant: **MONSANTO EUROPE S.A./N.V.**
**Avenue de Tervuren 270-272,**
**Letter Box 21**
**B-1150 Brussels (BE)**

(72) Inventor: **Janssens, Bonny Louis Jules**
**Haarbeekweg 2**
**B-3370 Boutersem (BE)**

(74) Representative: **Ernst, Hubert G. et al**
**Monsanto Services International S.A.**
**Letter Box 21**
**Avenue de Tervuren 270-272**
**B-1150 Brussels (BE)**

(54) **Substrate material for a biofilm and biofilm reactor.**

(57) A substrate material for a biofilm is disclosed for use in a biofilm reactor characterised in that the substrate material comprises a three-dimensional thermoplastic polymeric layer composed of a ground layer having a sheet structure with or without a multiplicity of perforations or openings, or having a net structure, and which ground layer is provided at least at one major side with a multiplicity of projections. Also the use is disclosed of the said substrate material as substrate material for the biofilm in a biofilm reactor. Furthermore, a biofilm reactor is disclosed containing the said substrate material for the biofilm, as well as a process for the purification of aqueous effluents containing organic waste by means of the said biofilm reactor. The substrate material and the biofilm reactor are suitable for use in systems for the purification of waste waters from agro-industries, food industries and beverage industries.

FIG.2.

EP 0 630 859 A1

This invention relates to a substrate material for use in a biofilm reactor. More particularly, this invention relates to a three-dimensional thermoplastic polymeric layer for use as substrate material for the biofilm in a biofilm reactor, to the use of a three-dimensional thermoplastic polymeric layer as substrate material for the biofilm in a biofilm reactor, and to a biofilm reactor containing the said substrate material. The substrate material and the biofilm reactor according to the invention are particularly suitable for use in a system for the treatment of aqueous effluents which contain organic waste. The present invention furthermore relates to a process for the purification of an aqueous effluent containing organic waste by the said biofilm reactor.

It is well known since several decades that aqueous effluents containing organic materials (hereinafter waste waters) can be purified to a certain extent by the action of micro-organisms. Many micro-organisms, indeed, are capable of degrading, under aerobic and/or anaerobic conditions, organic compounds and organic materials, (collectively termed herein organic waste) and, in this manner, to remove organic waste from or reduce its concentration in waste waters. Under aerobic conditions, organic carbon (i.e. carbon comprised in an organic compound) is biologically degraded yielding energy used by the micro-organismss for their metabolic functions, carbon dioxide, water and biomass material, whereas organic nitrogen (i.e. nitrogen comprised in an organic compound) is biologically transformed by a nitrification process via ammonia into nitrate and energy, which nitrate can, by a biologically denitrification process, be further transformed into molecular nitrogen.

Based on the degrading action of micro-organisms, many systems for the treatment of waste waters, have been developed (the terms treatment and purification are used interchangeably hereinafter). Some of these purification systems, particularly systems for the treatment of sewage, are in use at large scale for many years. The term "sewage" herein refers in particular to domestic waste waters and to aqueous discharges containing organic waste which can be biologically decomposed similarly easily. In these purification systems the micro-organisms are commonly present in the form of a so-called "biomass". Such biomass is composed of one species or - commonly - of multiple species of micro-organisms and extracellular products such as exopolysaccharides produced by the micro-organisms. A biomass typically includes micro-organisms such as bacteria, fungi, protozoa and algae. It may further contain higher organisms such as snails, worms, larvae and insects. The biomass can be present, depending on the type of purification system, in various forms. It can, for example, be present in the form of activated sludge, of particles suspended in the sewage, or as a layer - commonly termed biofilm - adsorbed by natural forces and processes or immobilised by known techniques on a substrate. The substrate can be formed by larger solid structures which are immobilised and subjected to a flow of the waste water passing through the purification system or which are kept moving, e.g. rotating, in the waste water. Such systems are commonly termed biofilm systems. The substrate can also be formed by small particles which are kept in suspension in the waste water. The latter system is commonly named a fluidised bed system.

Substrate materials which are commonly used in biofilm reactors include, inter alia, porous sand, porous stones e.g. lava stones, slag, ceramic Raschig-rings, wood cuttings, and various polymeric articles, particularly various types of polymeric Raschig-rings.

Typical examples of biofilter purification systems include percolating filters, trickling filters and rotating biological contactors. The thickness of a biofilm may vary considerably and commonly ranges from about 0.01 mm to several millimeters. Commonly a biofilm has a thickness of about 2 to 3 mm. It often contains an lower layer, adsorbed to or immobilised on the substrate, in which the microbiological degradation of organic waste essentially occurs, due to oxygen deficiency, through anaerobic processes, and an upper layer, in direct contact with the aqueous effluent and with oxygen from the air dissolved in it, in which the microbiological degradation essentially occurs via aerobic processes.

In a percolating filter (commonly termed immersed biofilter) the biofilm is adsorbed or fixed on an immobilised substrate which is immersed in the aqueous effluent. The waste water is passed through the filter system under the force of gravity and/or by means of a pumping device while air is pumped through the system to allow aerobic degradation processes to occur.

In a trickling filter system the biofilm is adsorbed or fixed on an immobilised substrate contained in a reactor vessel. The waste water is spread on top of the substrate material and passes under the force of gravity through the filter. As a result of the loose packing of the substrate material appropriate oxygen exchange can occur between the liquid layer on the biofilm and the air available in the interstices. Optionally, an additional air stream can be passed through the trickling filter.

In a rotating biological contactor ("RBC") the substrate material with an adsorbed or immobilised biofilm is forming or contained in a rotor which is installed horizontally. The rotor is partly immersed in the waste water and is rotated in such a way that the initially immersed part of the substrate material is brought into contact with the air for a certain period, determined by the rotation speed of the rotor, before it is again immersed in the waste water.

The micro-organisms, biomass and mechanisms involved in the degradation of organic waste in aqueous effluents, as well as the various types of biofilm-based purification systems and processes and their specific parameters, advantages, disadvantages and performances, have been extensively described and are well known in the art.

FR-A-2,565,579 for example describes a biofilm-based purification system for waste waters in which the biofilm is adsorbed on glassfibers which are arranged in the form of a cylindrical brush. The nature of the substrate material, its assembly to form a cylindrical brush, and the difficulties and costs related to the installation, maintenance and replacement of the brush in a purification system, makes this system technically complicated and expensive.

WO 91/01279 describes a biofilter for the treatment of sewage which contains several assemblies made from ribbons of plastic material which are essentially continuous and arranged in a spiral around two supporting members. The hanks of ribbons are arranged vertically in the container forming the biofilter and are immersed in the waste water to be treated.

EP-A-0,332,907 discloses a substrate material for use in trickling filters for the purification of sewage, which is composed of ribbons of polymeric material, superimposed in bunches and affixed to a central support. The ribbons are provided with a cord-like polymeric means which absorbs the tension in the strips and prevents the strips from losing their initial surface waves and from sticking together. In this manner, the ribbons are prevented from losing their interstices and as a result thereof their performance.

Having been optimised for years, current biofilm purification systems perform reasonably well. Nevertheless they still show disadvantages, related directly or indirectly to the substrate material for the biofilm. The disadvantages include, inter alia, the cost of the substrate material, technical difficulties to install and/or replace the substrate material, the need for complicated or expensive means to support or immobilise the substrate in the biofilter system, the need for reactor or container means to contain the substrate and the waste water during the purification process, difficulties related with the easy clogging of the interstices of the biofilter, difficulties related to the cleaning of clogged substrate material which cleaning is often almost impossible or economically unattractive for certain substrate materials, a disadvantageous ratio of, inter alia, specific surface of substrate : volume; of specific surface of substrate : weight; and of size of biofilm reactor : purifying performance.

The object of the present invention is to provide an alternative, improved substrate material for a biofilm for use in a biofilm reactor, to provide an improved biofilm reactor which is particularly suitable for use in systems for the treatment of aqueous effluents containing organic waste, and to provide a process for the purification of waste water.

DESCRIPTION OF THE INVENTION

Figure 1    is a pictoral view of a substrate material manufactured by CIM technique having multiple openings in the ground layer and blade-like projections at one major side of the ground layer.

Figure 2    is a pictoral view of a substrate material manufactured by CIM technique having a multiplicity of openings in the ground layer and grass-like projections at one major side of the ground layer.

Figure 3    is a schematically representation of a pilot scale trickling filter.

According to the present invention a substrate material for a biofilm for use in a biofilm reactor is provided, characterised in that the substrate material comprises a three-dimensional thermoplastic polymeric layer composed of a ground layer having a sheet structure with or without a multiplicity of perforations or openings or having a net structure, which ground layer is provided at least at one major side with a multiplicity of projections.

The thickness of the ground layer preferably ranges from about 0.5 mm to about 5 mm, more preferable from about 1 mm to about 3 mm. The length or depth of the projections preferably ranges from about 0.5 cm to about 5 cm, more preferably from about 1 cm to about 4 cm.

The projections may have various forms, including, for example, a cylinder -, a beam -, a beaker -, a bossed -, or a cone form which forms can have a convex, a concave or filled structure. Further forms of the projections include for example a pyramid form, a brush-like form, a blade-like form, a grass-like form, and the form of spikes.

The ground layer and the projections can be made from any extrudable or mouldable thermoplastic polymer, polymer mixture and polymer composition which optionally can contain further additives commonly used in the art such as, for example, plasticisers, stabilisers, fillers, and dyes, provided the polymer and polymer composition are essentially inert under the working conditions in the biofilm reactor. Suitable thermoplastic polymeric materials include polyolefins, polyvinyl chloride, polyvinyl esters such as polyvinyl acetate, styrene copolymers such as ABS blends, and blends or copolymers thereof. Other suitable polymeric materials include thermoplastic condensation polymers such as for example polyamides, polyesters and synthetic and natural rubbers. Preferably, the substrate is made from a polyolefin or polyolefin mixture, most preferably from polyethylene, high density polyethylene, low density polyethylene, polypropylene or a mixture or composition thereof. Suitable polymers, polymer mixtures and compositions are commercially readily available.

In one embodiment the substrate material is manufactured by known extrusion techniques, such as for example extrusion in combination with thermoforming and/or embossing techniques.

In another, preferred embodiment, the substrate material according to the invention is manufactured by injection moulding, most preferably by continuous injection moulding (herinafter CIM). The use of this technique ensures a firm bond between the ground layer structure and the projections of the three-dimensional moulded product. The CIM technique furthermore enables the manufacture, in a technically and economically attractive manner, of continuous strips of substrate material which allows the material to be wound up in rolls of a desired diameter for use as such in the biofilter. Thermoplastic polymeric materials and polymer compositions suitable for the manufacture of three-dimensional polymeric layers for use according to the present invention and methods for the manufacture of the substrate material, in particular via continuous injection moulding are well known in the art and for example described in detail in US 3,507,010 ; EP 92870128.3 ; DE 1,753,882 and DE 1,779,059.

In a preferred embodiment the ground layer is provided with a multiplicity of projections at one major side only. In such execution form the ground layer may optionally be laminated at the side opposite to the projections with a polymeric backing layer, and may optionally comprise in between a polymeric reinforcement layer and/or one or more polymeric adhesive layers ensuring a firm bond between the ground layer, the possible reinforcement layer, and the backing layer. Such three-dimensional structures and their manufacture are known in the art and described for example in EP 0,024,203 and EP 0,091,419.

In a highly preferred embodiment the substrate material according to the invention is a grass-like three-dimensional thermoplastic polymeric layer, manufactured by continuous injection moulding in the form of a long strip, having a ground layer of about 0.5 mm to 3 mm thickness, typically of about 1 mm to 2 mm thickness, with a multiplicity of perforations or openings, commonly of about 3 mm to 15 mm, typically of about 5 mm to 10 mm in diameter, and provided only at one major side with blade-like or grass-like projections of about 1 cm to 3 cm long, typically of about 1.8 cm long. Preferably the moulded product is made from polyethylene, more preferably from low density polyethylene, and is sufficiently pliable to allow the strips to be rolled up without any damage to their structure.

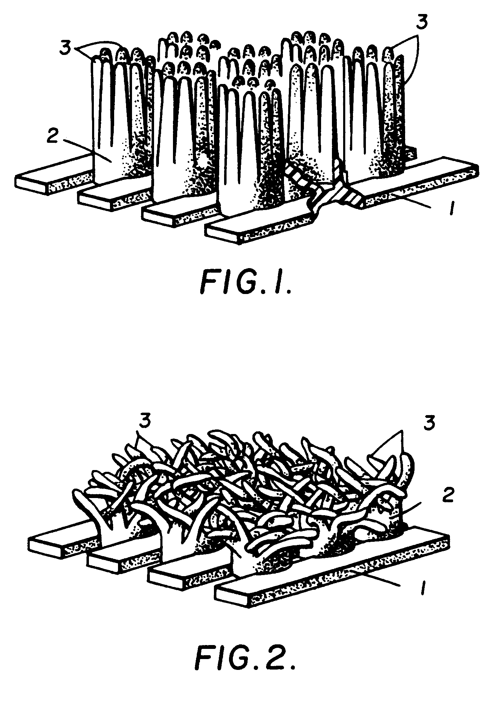

A typical, highly preferred, substrate material according to the invention is commercially available from Monsanto Company, e.g. as CIM Astroturf® material (Registered Trademark of Monsanto Company). This material shown in two embodiments in Figures 1 and 2, is a preferred shape for the substrate material of this invention. It is produced as a thermoplastic, three-dimensional, moulded grass-like product such as that shown in U.S. Patent 3,507,010. The moulded product has a relatively flat matrix formed of parallel strips or ribs (1) separated by hollow, circular clusters or buds (2) from which blade-like projections (3) extend. The clusters (2) do not abut one another. They are separated from one another to provide openings or holes in the matrix between adjacent clusters (2) and through the center of the hollow clusters (2) thus allowing easy flow of aqueous effluent and air through the substrate material. In a preferred embodiment the projections (3) extending from the clusters (2) are essentially vertical after being molded, as shown in Figure 1. In another embodiment the appearance of natural grass is imparted to the moulded product by texturing the molded material to disperse the tips of the projections (3) randomly.This texturing imparts a permanent crimp in the projections (3) whereby they remain bent or flattened with the tips dispersed randomly over the surface, as shown in Figure 2.

The biofilm substrate material according to the invention is suitable for use in biofilm reactors, particularly in trickling filters, in immersed-type biofilters, and in rotating biological contactors. The construction, the functioning and maintenance of biofilm reactors is well known to the skilled person and comprehensively described in the literature of the art. The construction, functioning and maintenance of a biofilm reactor containing a biofilm substrate material according to the present invention can be made essentially in an analogous manner.

In a further embodiment, the present invention provides the use of a three-dimensional thermoplastic polymeric layer, defined hereinbefore, as substrate material for the biofilm in a biofilm reactor.

It is a further embodiment of the present invention to provide a biofilm reactor which contains as substrate material for the biofilm a substrate material according to the present invention. In a preferred embodiment, the biofilm reactor of the present invention relates to a trickling filter. In another preferred

EP 0 630 859 A1

embodiment the biofilm reactor of the present invention relates to an immersed biofilter, and in still a further preferred embodiment to a rotating biological contactor.

In a particularly preferred embodiment, the present invention relates to a trickling filter in which the substrate material for the biofilm is a grass-like, three-dimensional thermoplastic polymeric layer having blade-like or grass-like projections at one side only of the ground layer as defined hereinbefore and manufactured from polyethylene through continuous injection moulding.

In such a trickling filter, the substrate material according to the invention is wound up in a roll, preferably with the projections directed towards the axis of the roll, which is installed in vertical position. By an appropriate device, known in the art, the waste water to be treated is applied, preferably spread homogeneously over the surface, on top of the roll of substrate material and flows under the force of gravity through the roll. On its way to the bottom the effluent is brought as a thin, aqueous film into intimate contact with the biofilm on the surface of the substrate on the one hand and with oxygen from the air, present in the interstices of the substrate, on the other hand. During the stay on the biofilm, which is initially grown on the substrate by known techniques, the micro-organismss degrade the organic waste, thus purifying the waste water. The chimney effect occurring in cylindrical trickling filters ensures an adequate air supply through the filter in the absence of any mechanical air flow providing device, which in fact constitutes an advantage of a trickling filter according to the invention. At the bottom of the substrate material, the aqueous effluent can be discharged or collected and further treated according to known methods, including e.g. by passing it through a settling container to enable possibly present biomass to settle before the supernatant aqueous effluent is discharged via a continuous overflow. Optionally the effluent, before or after staying in the settling container, can be recycled as such or mixed with fresh waste water over the trickling filter.

A trickling filter, according to the invention, can be realised through an extremely simple construction, essentially comprising a known pump device to supply the waste water, a roll of substrate material according to the invention, and a known device to collect the treated aqueous effluent which is provided with an overflow and optionally with pumping means enabling a partial or complete recycling of the effluent over the substrate material. Although the roll of substrate material can be enclosed in a, for example, cylindrical reactor, it can also, as a result of its inherent rigidity, be installed, preventing the roll from unrolling through a suitable known fixing means, put on a known supporting means, without any lateral walls. This way of installing the substrate material will advantageously enable an increased air flow through the biofilter unit via the outer lateral layer. It is furthermore advantageous for a good performance of the filter to spread the waste water in a homogeneous manner over the surface on the top of the roll of substrate. This can be realised by known means including for example a multiple perforated bottom plate of a container having a diameter slightly smaller than the diameter of the roll of substrate, a shower-like spray device and a horizontally rotating, perforated pipe. Furthermore, in order to reduce the effect of weather conditions such as direct sunshine, heat, frost, and wind, it may be advantageous to add to the roll of substrate, the diameter of which has been dimentioned in fuction of the required purification performances, one or more extra windings of the substrate material which are essentially not contacted with waste water. The extra winding(s) can act as an insulation layer which is shielding the inner part of the trickling filter from weather conditions which are unfavourable for good functioning of the filter.

An immersible biofilm reactor according to the present invention, for example an immersed biofilter for the treatment of sewage, can be realised by installing the substrate material of the present invention in a reactor vessel, i.e. a container provided with a supply of waste water to be treated, an air supplying device and an outlet for the treated aqueous effluent. The substrate material, if provided with projections at one major side only, can be installed in the biofilm reactor in the form of a wound up roll, in a vertical as well as in a horizontal position, prevented from unwinding by appropriate, known fixing means, or in the form of plates or panels, sized in accordance with the size of the bio-reactor and installed horizontally or vertically with all projections pointing in the same direction.

A rotating biofilm contactor according to the invention, may be realised, for example, by having a wound up roll of substrate material adequately provided with and connected to supporting means in the form of an axis and/or in the form of a supporting frame means at the outer side of the roll, thus forming a rotor. The rotor is installed essentially horizontally so that the substrate material is partly immersed in the waste water, and is rotated by known means. Through the rotation, the biofilm adsorbed on the substrate material is alternatively immersed in the waste water and brought into contact with the air, thus creating conditions for the micro-organisms of the biofilm which are suitable for the biological degradation of organic waste.

It is important that the interstices in the substrate material and in rolled up or stacked up substrate material in the biofilm reactor are sufficient in size and in number to allow the aqueous effluent and the air to readily flow through. A ground layer with too many or too small projections, or with projections on both major sides, or ground layers with projections at one major side and joined together through the other major

5

side, may, when utilised in a roll or stacked in the form of plates or panels to a volume , present a too dense structure preventing the aqueous effluent and air from passing readily through it. In such case it is advantageous to ensure that the windings, plates or panels are sufficiently separated from each other by a suitable known spacer device or installed in such way as to ensure adequate space between the subsequent substrate layers.

In a further embodiment the present invention relates to a process for the purification of an aqueous effluent containing organic waste by means of a biofilm reactor, which process comprises bringing waste water into contact with a biofilm on a substrate material present in the biofilm reactor, which substrate material comprises a three-dimentional, thermoplastic polymeric layer as defined earlier in the present invention. The purification is preferably carried out in the presence of air (oxygen) by means of a trickling filter, an immersed biofilter or a rotating biological contactor, more preferably a trickling filter.

The extent to which the waste water is purified is dependent on many parameters which are known in the art including for trickling and immersed biofilters, the size of the biofilter, the flow rate of the effluent, the retention time of the effluent on the biofilm, the kind of species or mixture of species of micro-organismss, the kind and amount of organic waste in the effluent, the degree to which the effluent has to be purified, the oxygen (air) supply to the biofilter, and the ratio of recycled waste water, if any, to fresh one. The optional value of these and other parameters can easily be determined by the skilled person via routine experiments.

The substrate material and biofilm reactor, according to the present invention, present significant advantages over known substrate materials and biofilm reactors charged with known substrate materials. The advantages include, inter alia, the provision of a biologically non-degradable substrate material with a very high specific surface per cubic meter of substrate material in rolled up as well as in stacked form, the provision of a substrate material which is not toxic to micro-organismss and on which micro-organismss can readily adsorb to form a biofilm. Further advantages include the fact that rolls of the substrate material can be sufficiently rigid to be self-supporting which removes the need of complicated or expensive supporting means to install the substrate in a biofilm reactor, as well as the readily availability of the polymeric materials and CIM produced substrate material at economically attractive costs. Still further advantages include the fact that when substrate material with projections at one major side only is used, normally no spacer means are required to ensure that interstices are present in sufficiently large number and size to enable adequate flow of waste water and air through the substrate unit. A roll or a stack of plates or panels of the substrate material is termed herein a (substrate) unit.

Still further advantages include the fact that the substrate material becomes not easily clogged, and that substrate units can easily be declogged by an air jet or a jet of high pressure water or waste water without dismantling the biofilter. Still further advantages include the high durability and robustness of the substrate material which avoids the need to renew it at regular intervals, the ease substrate units can be dismantled, cleaned with a jet of high pressure water and reused in the biofilter. Further advantages include the high purification performance of a biofilter equipped with substrate material of the invention, and the fact that aerobic and anaerobic areas can be present in the biofilm on the substrate. Still further advantages relate to the ease the substrate material can be installed in a biofilm reactor, the ease the substrate units can be dimensioned, e.g. by cutting plates or panels with a desired size from strips, by winding up strips of substrate to the desired diameter and by piling up plates, panels or rolls of substrate material to meet a desired volume or heighth.

The substrate material, according to the invention, is particularly suitable for use in biofilm reactors for the treatment of domestic waste waters and industrial waste waters containing organic waste which can be biologically degraded similarly easily. Such waste waters typically include waste waters from agro-industries, such as e.g. waste waters containing organic waste from dairy industries (e.g. milk and derived products), from pig-farms, and from slaughter-houses, and waste waters from the beverage and food industries such as e.g. breweries and fruit juice industries.

The invention is illustrated by the following examples in which a waste water has been purified by means of a trickling filter containing as substrate material according to the invention CIM Astroturf® (Registered Trademark of Monsanto) with vertical blade-like projections or grass-like projections, both having a specific surface of about 396 $m^2/m^3$ (calculated value).

6

General procedure :

An artificial waste water has been prepared containing :

| - glucose | 1,0 g/l |
|---|---|
| - pepton | 0,1 g/l |
| - yeast extract | 0,1 g/l |
| - $(NH_4)_2SO_4$ | 0,1 g/l |
| - $KH_2PO_4$ | 0,1 g/l |

which had a measured carbon : nitrogen : phosphorus (C:N:P) weight ratio of 100 : 7,7 : 4,4. Carbon (C) was measured as chemical oxygen demand (COD) with $K_2Cr_2O_7$ according to method NBN T91-201 (Belgisch Staatsblad 1974). Nitrogen (N) was measured as nitrate and as total nitrogen (Kjeldahl method) (TNK) according to the procedures NBN T91-255 and NBN T91-256 (Belgisch Staatsblad 1977). Phosphorus was measured via a known, standard colorimetric method.

The artificial waste water has been prepared in batches of about 800 l and stored during the experiments at about 5 to 10°C to prevent the growth of micro-organismss in the storage tank.

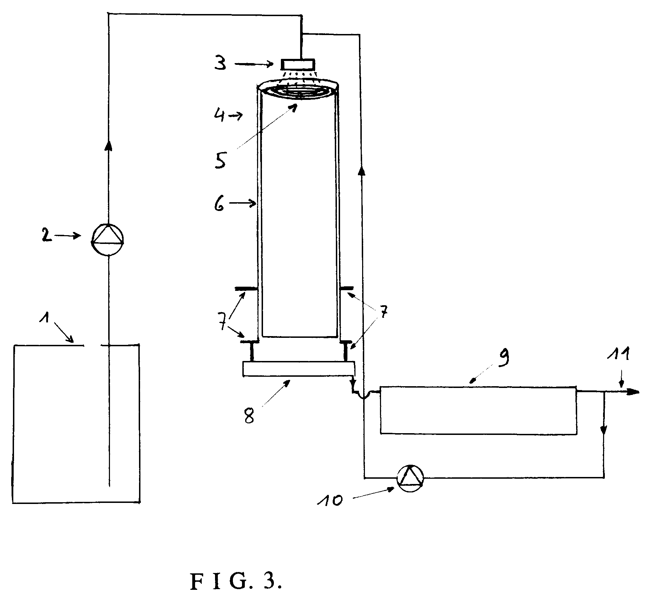

A trickling filter at pilot scale has been installed as shown in Figure 3 , comprising :

a storage tank (1) containing the artificial waste water stored at 5°C to 10°C, connected via a pump (2) to a waste water distributor (3), the biofilter (4) comprising a roll of substrate material (5) immobilised in a polyvinyl chloride pipe (6); supporting means (7), a collector (8) at the bottom side of the biofilter which is connected to a settling vessel (9) from which the waste water by means of a pump (10) can be partly or completely recycled over the filter, either as such or as a mixture with fresh waste water, and from which the treated waste water can be discharged via an over-flow (11). Samples for analyses were taken at pump (2) (i.e. fresh waste water) and at pump (10) (treated waste water). The temperature in the trickling filter was about 15°C.

The trickling filter has been inoculated with 0,5 l activated sludge from a sewage purification plant. The sludge was diluted with tap water and recycled for one week over the filter before the Examples were run.

The parameters of the trickling filter system are defined as follows, and the numeric values are presented in Tables 1 and 2 below.

- flow rate (Q) (in m³/h) :
  Q in. : flow rate of fresh waste water to the filter
  Q rec. : flow rate of recycled waste water to the filter
  Q total : sum of Q in + Q rec.
- hydraulic charge (q) (in m³/m²/h) :
  q : is the total flow rate per surface unit of the horizontal cross section of the substrate. In a highly charged trickling filter the hydraulic charge q is between 0,6 and 2 m³/m²/h and in a slightly charged trickling filter q is between 0,05 and 0,3 m³/m²/h.
- recycling factor (RF) :
  is indicating the number of times the waste water is recycled over the filter

  RF = 1 + R ( R = Q rec : Q in )

- apparent volumetric charge (B) (in kg BOD/m³/day) determined via the formula :

  BOD = F x 0.65 x COD

  wherein
  BOD = biological oxygen demand
  F = the factor indicating the degradability of the waste material (when 100% degradable F = 1).
  In Examples 1 to 6 F was assumed to be equal to 1.
  COD = chemical oxygen demand.
  In highly charged filters B ranges from 0,6 to 2 and in slightly charged filters from 0,07 to 0,6.
- weight of biomass (kg biomass per m³ substrate) :
  * biomass wet weight : was determined by differential weighing of a sample of substrate after leaking out for 10 minutes.

\* biomass dry weight (Xa) (in kg DS/m$^3$) was determined by differential weighing of a sample of substrate after drying at 110°C for a few days until constant weight. The amount of dry substance (DS) was found to correspond to about 5 % of the wet weight of the biomass.

- biomass charge (in kg BOD/kg dry biomass/day) :

is the volumetric charge B per amount dry substance DS of the biomass per cubic meter substrate.

biomass charge = B : Xa.

- biomass production (Px) (in kg DS) :

is the production of biomass per week, expressed in kg dry substance of biomass (DS). The parameter indicates only the amount of biomass released from the substrate and settled in the settling container.

- efficiency (E) is determined via the BOD values :

E (%) = 100 x BOD after treatment : BOD before treatment.

The results obtained in Examples 1 to 6 are summarised in Tables 2 to 6 below.

The data presented in Tables 2 to 6 demonstrate that substrate material according to the present invention is adequate as substrate material in a biofilm reactor, because :

- a biomass grows and is adsorbed on the substrate material,
- when waste water is treated in a trickling filter containing a biofilm on a substrate material according to the invention, the COD, nitrogen content and phosphorus content of the waste water is efficiently reduced.

**Table 1** : **Technical parameters of the pilot scale trickling filters**

| Example | Substrate Type (1) | Parameters of Trickling filter unit | | | | | | Recycling factor |
| | | Diameter of roll of substrate (in m) | Height of roll of substrate (in m) | Volume of substrate (in m³) | Flow rate of waste water in m³/h | | | |
| | | | | | Q total | Q in | Q rec | RF (2) |
|---|---|---|---|---|---|---|---|---|
| 1 | B L | 0,28 | 0,91 | 0,056 | 0,089 | 0,0034 | 0,085 | 26,4 |
| 2 | G L | 0,28 | 0,91 | 0,056 | 0,076 | 0,0033 | 0,072 | 22,9 |
| 3 | B L | 0,28 | 0,91 | 0,056 | 0,087 | 0,0062 | 0,081 | 14 |
| 4 | G L | 0,28 | 0,91 | 0,056 | 0,087 | 0,0062 | 0,081 | 14 |
| 5 | G L | 0,28 | 0,91 | 0,056 | 0,086 | 0,0071 | 0,079 | 12,2 |
| 6 | G L | 0,59 | 1,82 | 0,498 | 0,234 | 0,0068 | 0,227 | 34,4 |

Legend : (1)  B L : substrate with blade-like projections;  G L : substrate with grass-like projections.

(2)  In the pilot scale filter is the RF value high.  However, when in real purification systems normal heights of, for example, 5 m are used, the RF value will drop to normal e.g. to about 5.

**Table 2 : <u>Experimental data of pilot scale trickling filters</u>**

| Example | Hydraulic charge (in m³/m²/day) | Volumetric charge (1) B (kg/BOD/m³/day) | Biomass dry weight (Xa) (in kg DS/m³) | Biomass charge (B/Xa) (2) per week) | Biomass production (in kg DS |
|---------|--------|--------|--------|--------|--------|
| 1 | 34,69 | 1,51 | 6,99 | 0,22 | |
| 2 | 29,62 | 1,48 | 6,59 | 0,22 | |
| 3 | 33,91 | 2,13 | 22,86 | 0,09 | |
| 4 | 33,91 | 2,13 | 22,86 | 0,09 | |
| 5 | 33,7 | 4,78 | | | 0,085 |
| 6 | 20,54 | 0,53 | | | |

Legend : (1) the factor of degradability (F) is assumed to be 1 (i.e. the organic waste is 100% biodegradable).

(2) expressed in kg BOD/kg biomass (dry substance DS) / day.

EP 0 630 859 A1

## Table 3 : Evolution of the COD value of the waste waters

| Example | COD (1) | Time | | in | | hours | |
|---|---|---|---|---|---|---|---|
| | mg O$_2$/l | 0 (influent) | 2 | 24 | 72 | 96 | 168 |
| 1 | COD : | 1612 | 295 | 491 | 419 | 483 | |
| 2 | COD : | 1612 | 252 | 239 | 199 | 235 | |
| 3 | COD : | 1235 | 442 | 716 | | | |
| 4 | COD : | 1235 | - | 886 | | | |
| 5 | COD : | 2418 (2) | 651 | 1377 | 1266 | 1267 | 1789 |
| 6 | COD : | 2472 | | 289 | 127 | 133 | 794 |

Legend : (1) measured on unfiltered, treated waste water.

(2) mean value of 5 measurements.

EP 0 630 859 A1

**Table 4** : **Evolution of the nitrogen content of the waste waters**

| Example | Nitrogen (1) in mg/l | Time 0 | 2 | in 24 | 72 | hours 96 | 168 |
|---------|----------|--------|------|------|------|------|------|
| 1 | N : | 9,03 | 0,56 | 0,56 | 0 | 0 | |
| 2 | N : | 9,03 | 2,26 | 0 | 0 | 0 | |
| 3 | N : | 16,56 | 2,24 | | | | |
| 4 | N : | 16,56 | 3,57 | | | | |
| 5 | N : | 46,92 | | 2,82 | 7,14 | 11,45 | 8,99 |

Legend : (1) Nitrogen : measured as "nitrate-nitrogen" ("$NO_3$-N") (in mg N/l).

EP 0 630 859 A1

**Table 5** : **Evolution of the Phosphorus content of the waste waters**

| Example | Phosphorus in mg/l (1) | Time 0 | in 2 | hours 24 | 72 | 96 | 168 |
|---|---|---|---|---|---|---|---|
| 1 | P : | 19,6 | 9,8 | 13,0 | - | 16,3 | |
| 2 | P : | 19,6 | 9,8 | 14,7 | 7,3 | 5,7 | |
| 3 | P : | 20,0 | 4,2 | 9,2 | | | |
| 4 | P : | 20,0 | 5,9 | 9,2 | | | |
| 5 | P : | 14,8 | | 15,6 | 13,6 | 13,5 | 12,0 |

Legend : (1) Phosphorus : measured as "phosphate-phosphorus" ("$PO_4$-P") (in mg P/l).

**Table 6** : _Evolution of the Biomass on the substrate in the trickling filters_

| Example | Biomass Xa (kg DS/m$^3$) | Time 0 | 3 | in 17 | 25 | days (1) 31 | 45 | 59 |
|---------|--------------------------|--------|------|-------|------|-------|------|------|
| 1 | Xa | 0 | 0,06 | 2,09 | 1,84 | 6,99 | 5,73 | 6,87 |
| 2 | Xa | 0 | | | 2,28 | 5,17 | 6,15 | 6,59 |

Legend (1) : the growth of the biomass is initiated by inoculation of the trickling filter at day 3 with 0,5 liter active sludge from an industrial purification plant which is circulated, diluted with tap water, for one week over the filter before the artificial waste water is fed to the filter.

## Claims

1. A substrate material for a biofilm for use in a biofilm reactor characterised in that the substrate material comprises a three-dimensional thermoplastic polymeric layer composed of a ground layer having a

14

sheet structure with or without a multiplicity of perforations or openings, or having a net structure, and which ground layer is provided at least at one major side with a multiplicity of projections.

2. The substrate material of claim 1 which is manufactured by injection moulding.

3. The substrate material of claim 2 which is manufactured in strips by continuous injection moulding.

4. The substrate material of claim 1 which is manufactured by extrusion, optionally in combination with thermoforming and/or embossing techniques.

5. The substrate material of any of claims 1 to 4 in which the thermoplastic polymer is a polyolefine or mixture of polyolefines selected from polyethylene, low density polyethylene, high density polyethylene and polypropylene.

6. The substrate material of claim 5 which is a grass-like three-dimensional thermoplastic polymeric layer manufactured by continuous injection moulding in the form of a long strip comprising a ground layer of 0,5 mm to 5 mm thickness with a multiplicity of perforations or openings which is provided one major side only with blade-like or grass-like projections of 0,5 cm to 5 cm length or depth.

7. The substrate material of claim 5 which is a three-dimensional, thermoplastic polymeric grass-like layer manufactured from low density polyethylene by continuous injection moulding, having a relatively flat matrix formed of parallel strips or ribs separated by hollow, circular clusters or buds which do not abut one another and from which essentially vertical blade-like projections or grass-like projections extend, the clusters being separated from one another to provide openings or holes in the matrix between the adjacent clusters and through the center of the hollow cluster.

8. The use of a substrate material defined in any of claims 1 to 7 as substrate material for the biofilm in a biofilm reactor.

9. A biofilm reactor comprising as substrate material for the biofilm a substrate material defined in any of claims 1 to 7.

10. The biofilm reactor of claim 9 which is selected from a trickling filter, an immersed biofilm and a rotating biological contractor.

11. The biofilm reactor of claim 9 which is a trickling filter containing the substrate material defined in claim 7 as substrate material for the biofilm.

12. A process for the purification of an aqueous effluent containing organic waste by means of a biofilm reactor comprising bringing waste water into contact with a biofilm on a substrate material present in the biofilm reactor which substrate material comprises a three-dimensional, thermoplastic polymeric layer as defined in any of claims 1 to 7.

13. The process of claim 12 in which the waste water comprises domestic waste water or an aqueous discharge containing organic waste which can be biologically decomposed similarly easily.

14. The process of claim 13 in which the waste water is an aqueous effluent containing organic waste from an agro-industry, a food industry or beverage industry.

FIG.1.

FIG.2.

F I G. 3.

European Patent Office

**EUROPEAN SEARCH REPORT**

Application Number

EP    93 87 0116

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 024 203 (MONSANTO COMPANY)<br>* page 3, line 31 - page 4, line 12 *<br>* page 5, line 1 - line 21 *<br>* page 6, line 25 - line 32 *<br>* page 7, line 29 - line 30; figure 1 * | 1-7 | C02F3/04<br>C12N11/08 |
| D,Y | | 8-14 | |
| D,Y | WO-A-9 101 279 (COFIDO SOCIETE COOPERATIVE)<br>* abstract; figure 4 *<br>* page 1, line 13 - line 21 * | 8-14 | |
| X | DATABASE WPI<br>Week 8730,<br>Derwent Publications Ltd., London, GB;<br>AN 87-209691<br>& JP-A-62 136 296 (IIDA K) 19 June 1987<br>* abstract * | 1,5,8 | |
| A | US-A-3 729 364 (DOLEMAN ET AL.)<br>* figures 8,9,20 * | 1-8 | |
| D | & DE-A-1 753 882 | | TECHNICAL FIELDS SEARCHED (Int. Cl.5) |
| A | DE-A-3 514 817 (LINDE AG)<br>* page 4, line 18 - page 5, line 33 * | 1,5,8 | C02F<br>C12N |
| D,A | EP-A-0 332 907 (NORDDEUTSCHE SEEKABELWERKE AKTIENGESELLSCHAFT) | | |
| A | WO-A-8 607 617 (NORDELL) | | |

The present search report has been drawn up for all claims

| Place of search | Date of completion of the search | Examiner |
|---|---|---|
| BERLIN | 26 NOVEMBER 1993 | CEDER O. |